# EUROPEAN PATENT SPECIFICATION

(11) **EP 4 489 920 B1**
(45) Date of publication and mention of the grant of the patent: **31.12.2025**
(21) Application number: 23713508.2
(22) Date of filing: 02.03.2023
(51) Int. Cl.: B05C 1/06, A45D 34/04, A45D 40/26, A61F 13/38, A61M 35/00, B05C 17/00, B65D 47/42, B65D 75/00, B65D 77/24, B65D 81/32

(54) **PRODUCT DELIVERY DEVICE SYSTEM AND METHODS**
PRODUKTAUSGABEVORRICHTUNGSSYSTEM UND -VERFAHREN
SYSTÈME ET PROCÉDÉS DE DISPOSITIF DE DISTRIBUTION DE PRODUIT

(30) Priority: 10.03.2022 US 202263318537 P
(43) Date of publication of application: 15.01.2025
(73) Proprietor: Professional Disposables International Inc., Woodcliff Lake, NJ 07677 (US)
(72) Inventor: OSHINSKI, Matthew, Oak Ridge, NJ 07438 (US); CAHALAN, Gerard, Anthony, Milford, CT 06776 (US)
(74) Representative: Barker Brettell LLP
(86) International application number: PCT/US2023/014330
(87) International publication number: WO 2023/172427

(56) References cited:
- WO-A1-2009/157578
- US-A1- 2014 366 485
- US-A1- 2016 257 470
- US-A1- 2018 333 566

## Description

### CROSS-REFERENCE TO RELATED APPLICATIONS

This application claims priority to and the benefit of U.S. Provisional Patent Application Serial No. 63/318,537, filed March 10, 2022.

### FIELD

The disclosed subject matter relates to a product delivery device system and methods.

### BACKGROUND

Generally, devices and methods exist for dispensing and applying a product to a surface. For example, paint can be generally applied to a wall with a paint brush, shoe polish can be applied to a shoe with a cloth, and toothpaste can be applied to a toothbrush for cleaning teeth. Such products and devices can be messy and unfortunately spill when presented as separate units. In the medical field, it is common practice to prepare a patient for surgery by applying a fluid product, such as an antiseptic solution, to a target body portion with a device that must be activated prior to use. One of the many problems associated with some typical conventional product or fluid delivery devices is the inclusion of an ampoule that needs to be broken in order to release its fluid contents, which brings about risks such as occlusion of the device and loose glass contacting the patient's skin.

To overcome at least such problems in the medical field, fluid delivery devices have been designed that use components having sealable membranes rather than ampoules. One drawback of such devices is that they tend to employ complex levers or push button actuation, each of which requires a high degree of user effort and exertion of high activation forces and can require two hands to operate such devices. Such force is not optimal for the physical capabilities of all user group populations and such devices can be cumbersome. Such devices are not ergonomically designed for the end user, as such designs induce extensive stress or fatigue upon the end user during system activation. Further, traditional devices that employ a gravity-fed antiseptic to drain from an ampoule or have a membrane sealed bottle that drains into the foam applicator prior to patient application, can require up to a 60 second waiting time from activation of the device to the device being available to clean the skin of a patient.

Thus, there remains a continued need for an improved product delivery device system and methods. The presently disclosed subject matter satisfies these and other needs. Embodiments of the disclosed subject matter provide a device system and method that utilize a device that can release a product, such as an antiseptic onto the skin of a patient or release a product for application to a surface. Further, the device system and disclosed methods require minimal activation force due to the employment of a unique device and packaging system for a ready-to-use applicator design, and are thereby optimal for the physical capabilities of all target user group populations and ergonomically designed for the end user. Further, the device system and method require no waiting time for use of the device, unlike traditional devices mentioned above. Finally, the disclosed subject matter is readily adaptable to accommodate any desired volume of fluid for delivery, is designed to provide any desired tint color/concentration to the fluid being delivered, and avoids any accidental activation issues of prior conventional devices since the device is delivered in a ready-to-use condition.

US2014366485A1 discloses applicators for applying a solution or other composition (e.g., an antiseptic composition) to skin of a patient. The applicator may include a container body having a proximal end, a distal end, and a frangible member disposed at the distal end of the body. The applicator includes a first position where the container body and frangible member are a unitary piece (e.g., blow molded as a single piece), and a second position when the frangible member is rotated relative to the body to irreversibly break a weak point therebetween, releasing the composition in the hollow body through an opening. A porous applicator head may be positioned adjacent the distal end of the body and frangible member, so that the composition flows out the body, through the opening, and onto the head. The applicator may be formed in a blow-fill-seal process for improved sterility and ease of manufacture.

US2016257470A1 discloses an applicator package that may include a pouch formed from one or more pliable membranes, the pouch comprising a first compartment and a second compartment, the first compartment being separated from the second chamber by a sealing juncture, a material sealed within the first compartment and an applicator having an applicator end in a handle end sealed within the second compartment. The membrane is formed from a material and has a thickness so as to allow the membrane to be manually punctured by the applicator end of the applicator for insertion of the applicator end into contact with the material in the first compartment.

WO2009157578A1 discloses a simple and versatile dropper which can preserve a medical fluid, an antiseptic solution, a cosmetic solution, or the like, for a long term. The simple and versatile stopper consists of a soft and hollow swelled portion and a flexible tubular container, wherein one or more thin glass ampules filled hermetically with a selected liquid are contained in the tubular container, a cavity through which a glass ampule cannot pass is provided as a stopper near the joint of the swelled portion and the tubular container with the recess thereof facing toward the inside of the tubular container, and a cotton ball is fixed to the opening of the tube so that a solution can be preserved for a long term.

US2018333566A1 discloses a fluid delivery device and method is provided, comprising a handle, a plate, a foam applicator coupled to a bottom surface of the plate and impregnated with an antiseptic solution. A lid member protects the foam applicator from an external environment and defines at least one aperture therein, wherein the lid member is coupled to the plate to form a breakable seal between the plate and the lid member. A tray member, having a bottom surface and at least one sidewall to define a volume therein, contains the foam applicator. The foam applicator is hermetically sealed from the external environment by the lid member, plate and tray member. The lid member is detachable from both the plate and the tray to release the foam applicator from the fluid delivery device and is in a ready-to-use condition without further activation.

### SUMMARY

The present invention provides a product delivery device system, a method of manufacturing a product delivery device system and a method of using a product delivery device system as defined in the appended claims. Optional features are defined in the dependent claims.

The purpose and advantages of the disclosed subject matter will be set forth in and are apparent from the description that follows, as well as will be learned by practice of the disclosed subject matter. Additional advantages of the disclosed subject matter will be realized and attained by the devices particularly pointed out in the written description and claims hereof, as well as from the appended drawings.

To achieve these and other advantages and in accordance with the purpose of the disclosed subject matter, as embodied and broadly described, the disclosed subject matter includes a product delivery device system. The product delivery device system comprises a product delivery device having a handle with a first end, a second end, and a cavity defined therein between the first end and the second end, a product disposed within the cavity, a foam applicator pad coupled to the first end of the handle and in fluid communication with the cavity to receive the product, a sealing member disposed on an external surface of the handle and located between the first end and the second end of the handle; and a packaging to house the product delivery device therein, the packaging having a first chamber and a second chamber in a delivery condition, wherein the first chamber houses the first end of the handle and the second chamber houses the second end of the handle, wherein the first chamber and second chamber are separated by a first breakable hermetic seal and the sealing member; wherein the first end of the handle is isolated in the delivery condition from the second end of the handle to contain the product within the cavity of the handle and the first chamber in the delivery condition; wherein the first seal is breakable to release the first end of the handle from the package and the product delivery device is in a ready-to-use condition without further activation.

In accordance with another aspect of the disclosed subject matter, a method of manufacturing a product delivery device system, comprising providing a product delivery device system is provided. The method includes a product delivery device having a handle with a first end, a second end, and a cavity defined therein between the first end and the second end, a foam applicator pad coupled to the first end of the handle and in fluid communication with the cavity to receive the antiseptic solution, a sealing member, disposed on an external surface of the handle, located between the first end and the second end of the handle; and providing a packaging to house the product delivery device therein; forming a second chamber by sealing the second end of the handle with a first breakable seal disposed along the sealing member and along an interior of the packaging and with a segment of a perimeter seal; partially sealing the packaging around the first end of the handle along another segment of the perimeter seal to form a first chamber separate from the second chamber, wherein a portion of the first chamber proximate the foam applicator pad is unsealed; injecting product into the cavity of the handle through the unsealed portion of the first chamber; and sealing the portion of the first chamber proximate the foam applicator pad to hermetically seal the first chamber from the second chamber and an external environment in a delivery condition; wherein the first chamber is isolated from the second chamber in the delivery condition by the first breakable seal and at least a portion of the perimeter seal and wherein, upon breaking the first breakable seal and at least the portion of the perimeter seal, the product delivery device is in a ready-to-use condition without further activation.

In accordance with yet another aspect of the disclosed subject matter, a method of using a product delivery device system is provided, comprising providing a product delivery device system including a product delivery device in a ready-to-use condition without further activation, the device having a handle with a first end, a second end, and a cavity defined therein between the first end and the second end, a product disposed within the cavity, a foam applicator pad coupled to the first end of the handle and in fluid communication with the cavity to receive the product, a sealing member, disposed on an external surface of the handle, located between the first end and the second end of the handle, and a packaging to house the product delivery device therein, the packaging having a first chamber and a second chamber in a delivery condition, wherein the first chamber houses the first end of the handle and the second chamber houses the second end of the handle, wherein the first chamber and second chamber are separated by a first breakable hermetic seal and the sealing member, wherein the first end of the handle is isolated in the delivery condition from the second end of the handle to contain the product within the cavity of the handle and the first chamber; breaking the first seal at the sealing member to fluidly couple the first and second chambers of the packaging; releasing at least the first end of the handle from the packaging; and applying the product from product delivery device with the applicator foam pad.

In accordance with another aspect of the disclosed subject matter, a product delivery device system is provided, comprising a product delivery device having a handle with a first end, a second end, and a cavity defined therein between the first end and the second end, a product disposed within the cavity, a foam applicator pad coupled to the first end of the handle and in fluid communication with the cavity to receive the product, a sealing member disposed on an external surface of the handle and located between the first end and the second end of the handle; and a packaging to house at least a portion of the product delivery device therein, the packaging having a first chamber in a delivery condition, wherein the first chamber houses the first end of the handle and includes a breakable seal to release the first end of the handle from the package and the product delivery device is in a ready-to-use condition without further activation.

It is to be understood that both the foregoing general description and the following detailed description and drawings are examples and are provided for purpose of illustration and not intended to limit the scope of the disclosed subject matter in any manner.

The accompanying drawings, which are incorporated in and constitute part of this specification, are included to illustrate and provide a further understanding of the devices of the disclosed subject matter. Together with the description, the drawings serve to explain the principles of the disclosed subject matter.

### BRIEF DESCRIPTION OF THE DRAWINGS

The subject matter of the application will be more readily understood from the following detailed description when read in conjunction with the accompanying drawings, in which:
FIG. 1 is a top view of a product delivery device system according to the disclosed subject matter.
FIG. 2A is a top view of the product delivery device of FIG. 1, according to the disclosed subject matter.
FIGS. 2B-2C are perspective views of the product delivery device of FIG. 2A.
FIG. 3 is a perspective view of the product delivery device system of FIG. 1, according to the disclosed subject matter.
FIGS. 4A-4D depict a method of manufacturing and using the product delivery device system of FIG. 1, according to the disclosed subject matter.
FIG. 5A is an internal cross-sectional view of a step in the manufacture of the product delivery device system, according to the disclosed subject matter.
FIG. 5B is an internal plan view of a step in the manufacture of the product delivery device system, according to the disclosed subject matter.
FIG. 6 is an internal cross-sectional view of a step in the manufacture of the product delivery device system, according to the disclosed subject matter.
FIG. 7A and FIG. 7B are a top perspective view and a back perspective view of the plate of the product delivery device respectively, according to the disclosed subject matter.
FIG. 8 is an internal cross-sectional view of a metering device, according to the disclosed subject matter.
FIG. 9A is a perspective view of a monolithic foam applicator of the product delivery device of FIG. 2A, according to the disclosed subject matter.
FIG. 9B is a perspective view of an embodiment of the foam applicator having two sections, according to the disclosed subject matter.
FIG. 9C is a perspective view of another embodiment of the foam applicator having two sections, according to the disclosed subject matter.
FIG. 9D is a perspective view of an embodiment of the foam applicator having perforations, according to the disclosed subject matter.
FIG. 9E is a perspective view of an embodiment of the foam applicator having a two section laminate, according to the disclosed subject matter.
FIG. 10A is a perspective view of the product delivery device system in a delivery condition, according to the disclosed subject matter.
FIG. 10B is a perspective view of the product delivery device in a use condition, according to the disclosed subject matter.
FIGS. 11A and 11B is a top and perspective view of the product delivery device system in a delivery condition and a use condition respectively, according to the disclosed subject matter.
FIGS. 12 is an exemplary product formulation used with the product delivery device system, according to the disclosed subject matter.
FIGS. 13A and 13B is a side and an internal cross-sectional view of the product delivery device system in a delivery condition according to an embodiment of the disclosed subject matter.
FIGS. 14A and 14B is a side and perspective view of the product delivery device system during manufacture and in a delivery condition respectively, according to an embodiment which does not form part of the invention.
FIG. 15 is a perspective view of the product delivery device system, according to another embodiment which does not form part of the invention.
FIG. 16 is a perspective view of the product delivery device having a gasket, according to an embodiment of the disclosed subject matter.

### DETAILED DESCRIPTION

Reference will now be made in detail to embodiments of the disclosed subject matter, an example of which is illustrated in the accompanying drawings. The disclosed subject matter will be described in conjunction with the detailed description of the system.

In accordance with the disclosed subject matter, a delivery device system is provided. The product delivery device system comprises a product delivery device having a handle with a first end, a second end, and a cavity defined therein between the first end and the second end, a product disposed within the cavity, a foam applicator pad coupled to the first end of the handle and in fluid communication with the cavity to receive the product, a sealing member disposed on an external surface of the handle and located between the first end and the second end of the handle; and a packaging to house the product delivery device therein, the packaging having a first chamber and a second chamber in a delivery condition, wherein the first chamber houses the first end of the handle and the second chamber houses the second end of the handle, wherein the first chamber and second chamber are separated by a first breakable hermetic seal and the sealing member; wherein the first end of the handle is isolated in the delivery condition from the second end of the handle to contain the product within the cavity of the handle and the first chamber in the delivery condition; wherein the first seal is breakable to release the first end of the handle from the package and the product delivery device is in a ready-to-use condition without further activation.

A method of using and manufacturing the product delivery device system described above is also disclosed. The details of the method of using and manufacturing the device system will be described in detail in conjunction with the features of the product delivery device.

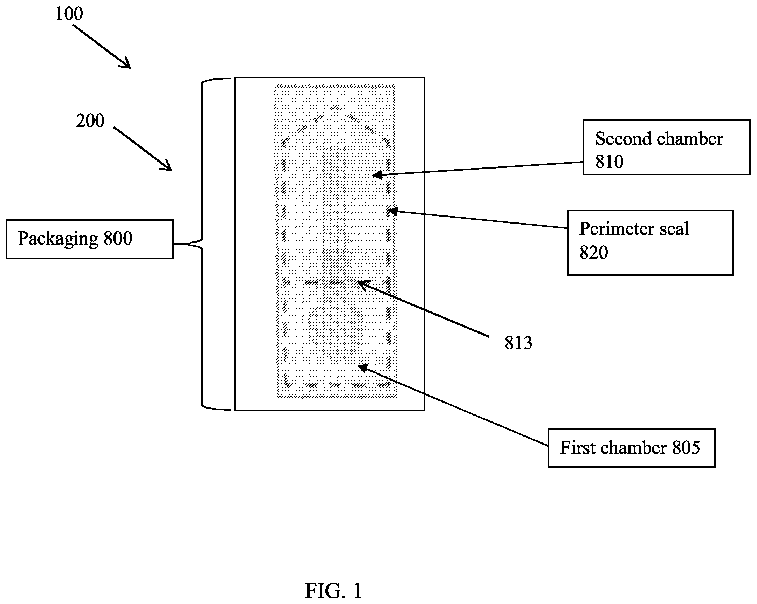

Solely for purpose of illustration, an embodiment of a product delivery device system 100 having a product delivery device 200 disposed within packaging 800 is shown in FIG. 1. The examples herein are not intended to limit the scope of the disclosed subject matter in any manner.

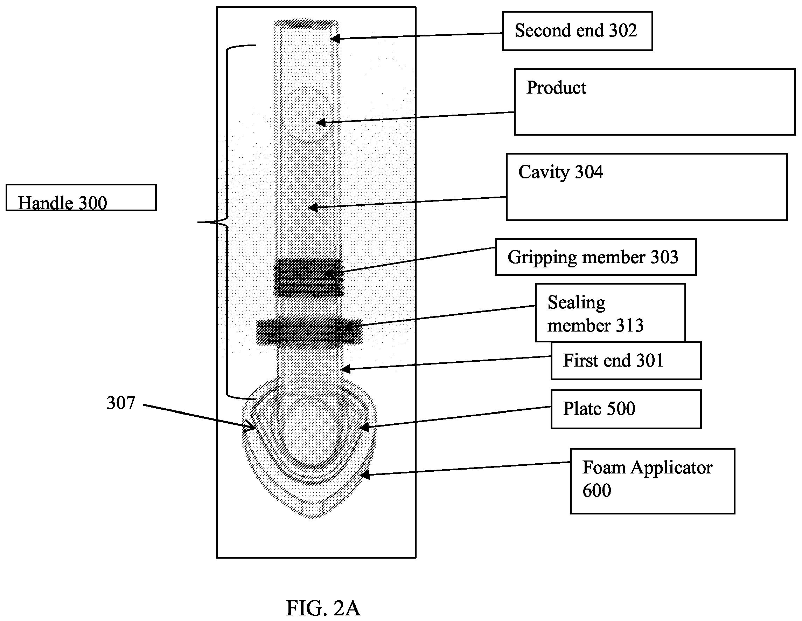

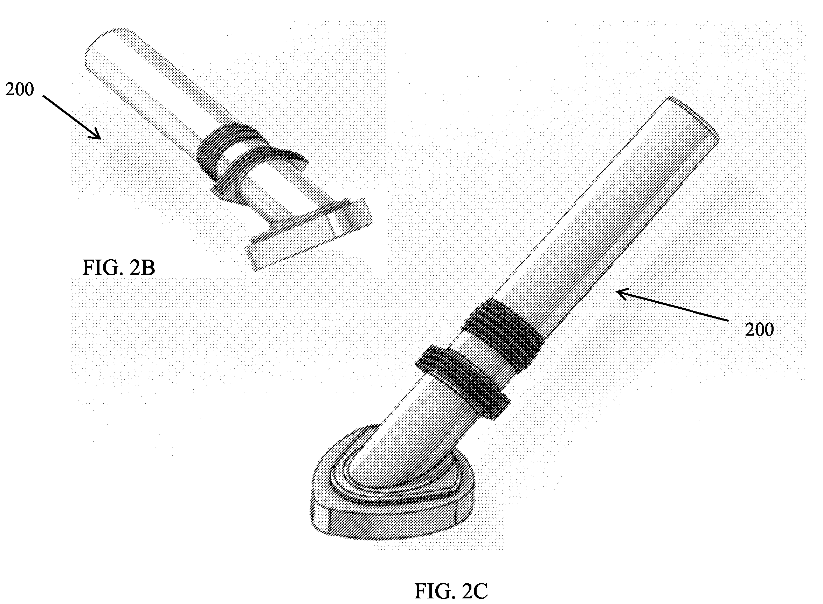

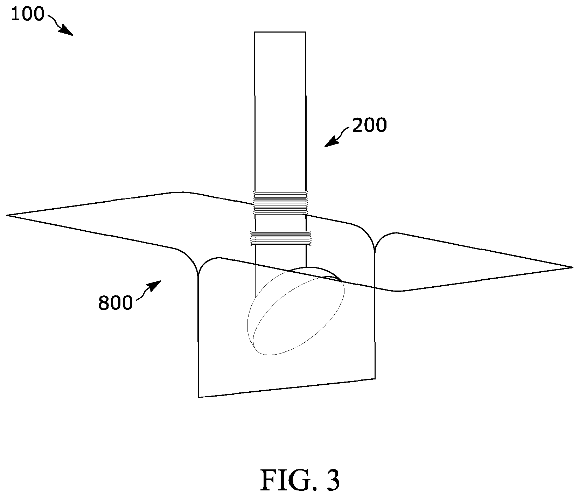

FIG. 2A depicts magnified view of the product delivery device of FIG. 1 without the packaging. FIGS. 2B-2C are perspective views of the product delivery device of FIG. 2A. Particularly, and as illustrated in FIG. 2A, the product delivery device 200 includes a handle 300 with a sealing member 313, a foam applicator 600, and a product disposed within a cavity of the handle 300. The product delivery device 200 is housed within the package 800 in a delivery condition. The product delivery device 200 can be removed from the package 800, as shown in FIG. 3 and further discussed herein.

The package or packaging 800 has a first chamber 805 and a second chamber 810 in a delivery condition, as shown in FIG. 1. The first chamber houses the first end 301 of the handle which can include the foam applicator 600 and the second chamber 810 houses the second end 302 of the handle. The first chamber and second chamber are separated by a first breakable hermetic seal 813 that seals with and to the sealing member 313 of the handle 300 and seals walls of the package 800 together along the seal 813. The first seal 813 is breakable to release the first end of the handle from the package 800 such that the product delivery device 200 is in a ready-to-use condition without further activation of the device. The seal 813 can be a transverse seal in relation to the longitudinal length of the handle 300. The first chamber and second chambers are separated along the sealing member 313 in the embodiment of FIG. 1-4D. The first chamber and the second chamber are openable independently from each other along portions of the perimeter seal 820.

The first chamber and the second chamber can be created by the walls of the package 800 and with seals. The seals can be disposed along an interior surface of the packaging. In one embodiment, the package material comprises at least a sheet of material that is fastened together by way of seals or folds to create the first and second chamber.

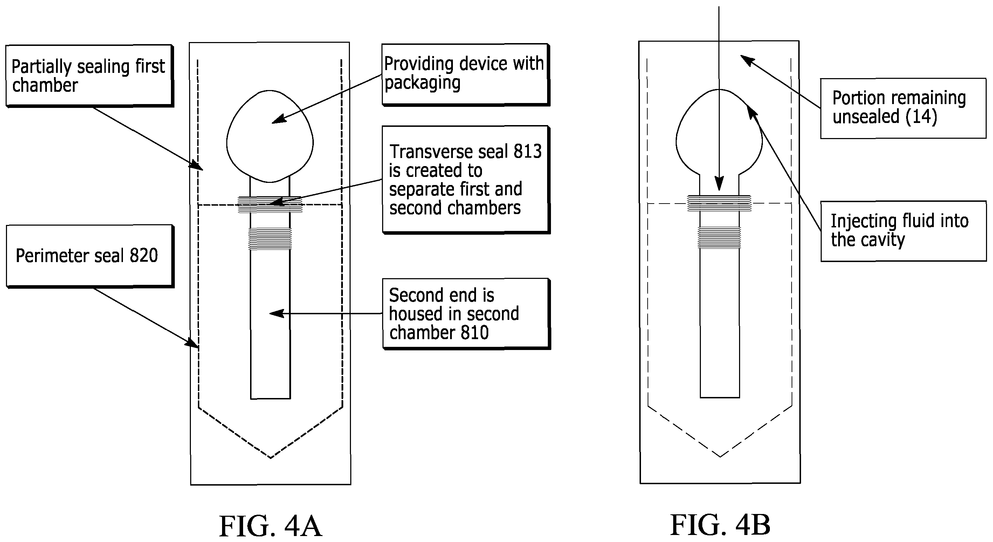

FIG. 4A-4D depicts an example process in which the package system can be manufactured. In FIG. 4A, a product delivery device 200 is provided as further discussed herein. As shown in FIG. 4A, a packaging 800 houses the product delivery device therein. The second chamber is formed by sealing the second end of the handle 300 along at least a segment of a perimeter of the package about the device as represented by perimeter seal 820. The second chamber is also sealed, along the transverse segment of the package at the sealing member 313 as represented by seal 813. The first chamber is formed by partially sealing the packaging around the first end of the handle along another segment of the package perimeter such that a portion of the first chamber proximate the foam applicator pad is unsealed, as shown in FIG. 4A.

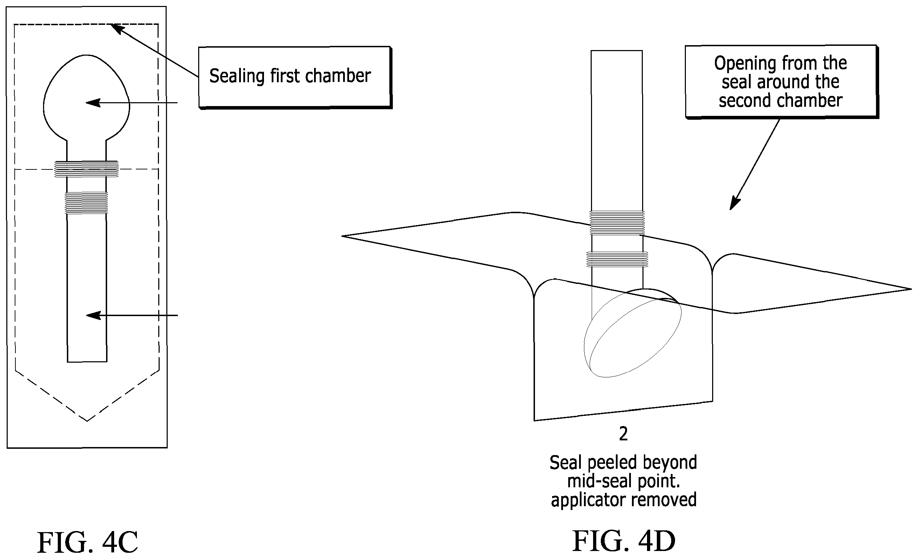

As shown in FIG. 4B, medium or solution is injected into a cavity of the handle through the unsealed portion of the first chamber, as further discussed herein. In FIG. 4C, the portion of the first chamber proximate the foam applicator pad is sealed to completely seal the first chamber such that the first chamber is separate from the second chamber. As such, the first chamber is isolated from the second chamber in a delivery condition at least by the first breakable seal at the transverse segment of the package at the sealing member 313. As shown in FIG. 4D, a first and second breakable seal, such as the perimeter seal and the transverse seal, can be broken such that the product delivery device is releasable in a ready-to-use condition upon removal without further activation. Prior to the disruption of the seals, the first end of the handle is isolated in the delivery condition from the second end of the handle to contain the solution within the cavity of the handle and the first chamber. The seals can comprise any suitable seal and method of sealing, such as but not limited to at least one of glue, adhesive, laminate, or ultrasonic weld. In one embodiment, the product delivery device can be secured with the foam pad in an upright position and a foil packaging can be sealed around the device leaving an end of the packaging adjacent the foam pad unsealed. As such, a heat sealing tool can surround the device and the foil like a clamshell, creating the perimeter and transverse seals except for the top seal adjacent the applicator foam pad. During such process, any excess air is pressed out of the second chamber of the packaging. Alternatively, the seals can be manufactured with multiple overlapping seals. Once the device is injected with product, a final seal as shown in FIG. 4C can hermetically seal the first chamber about the applicator foam pad. The seal can be an indicator of the chamber that includes the foam pad that is pre-saturated. As the first chamber is sealed at the final segment of the perimeter seal, a tool can furthermore compress the applicator foam pad into a compressed state or alternatively provide vacuum capabilities as discussed herein.

The product delivery device system 100 can deliver several different products, such as products with different flow rates and viscosities. For purposes of example, and not limitation, the product can include substances suitable for medical field application such as antiseptic solutions like chlorhexidine gluconate, isopropyl alcohol, povidone-iodine (PVP-I), and povidone-iodine with isopropyl alcohol (PVP-IPA), as discussed herein. The product can furthermore include substances outside the medical field such as make up, lotions, sunscreen, polishes, paint materials, detergent, soap, cleaners, toothpaste, and food products. Any flowable product can be used with the product delivery device and is contemplated herein, including a powder or packaged substance which requires rehydration or dilution.

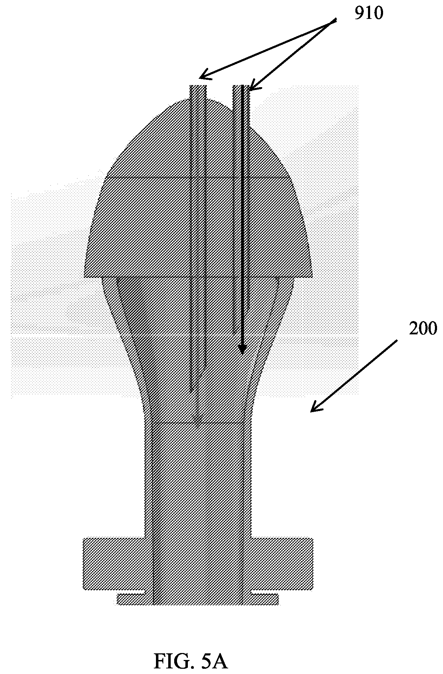

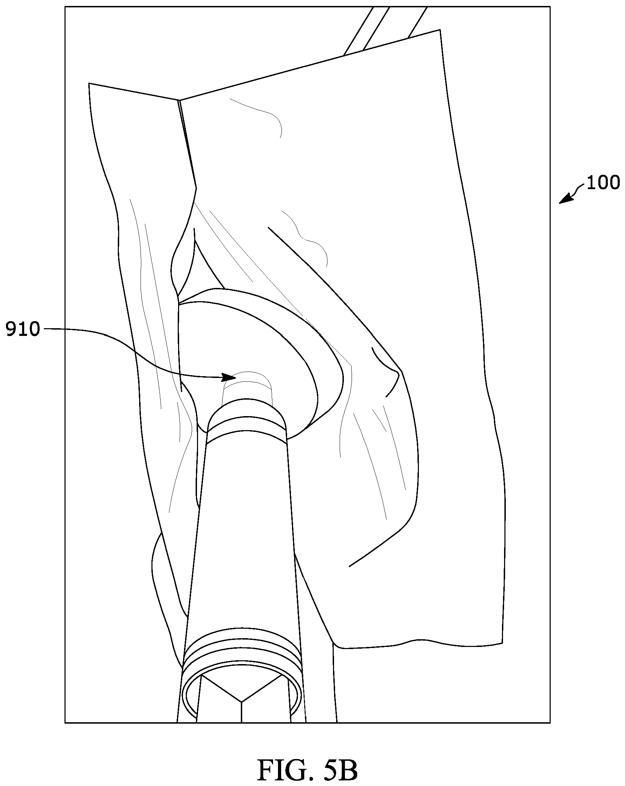

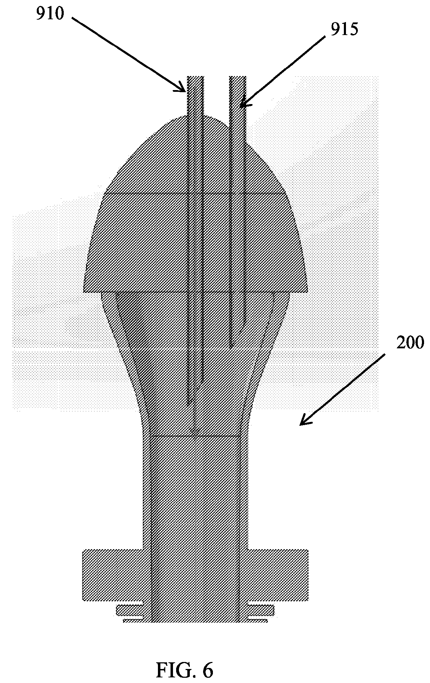

The product can be injected into the product delivery device 200 in any suitable manner. As shown in FIG. 4B, the product is injected into the device by way of the unsealed portion of the first chamber, as shown in FIG. 4B. In one embodiment, the product is injected into a cavity of the handle using one or more needles which puncture the foam applicator pad to access the cavity of the handle. FIG. 5A depicts an example of two needles inserted through the foam applicator pad to access a cavity within the handle of the device. The product can be delivered through the one or more needles 910 to fill a predetermined volume of the cavity with product for later administration. Any number of suitable needles can be used to inject the product into the device, as contemplated herein. FIG. 5B depicts an example of one needle inserted through the foam applicator pad to access a cavity within the handle of the device, while in the packaging 800. The needles can puncture the foam applicator pad to access the cavity of the handle, as further discussed herein. FIG. 6 depicts another example of a first needle 910 for injecting product into the device as shown by the arrow into the device 200 and a second needle for siphoning or venting air out of the device as shown by the arrow out of the device 200 for efficient filling. Any number of suitable needles can be used to inject the product into the device and venting air out of the device, as contemplated herein. In other embodiments, a single dual channel needle can be used that has a dual functionality of injecting along a first side and venting along a second side. With the injecting and venting, such actions can be conducted simultaneously or on time delay. Alternatively or additionally, the cavity of the handle can naturally vent upon injection of product therein, such as with the embodiment of FIG. 5B.

In another embodiment, the product can be inserted into the product delivery device prior to the foam applicator pad being affixed to the handle. With such embodiment, the product can be filled into the handle before the product delivery device is inserted into the package.

For purposes of example only, an embodiment directed to a product delivery device system is described with a product delivery device containing an antiseptic solution therein. The product or fluid delivery device can be used for skin cleansing, such as cleaning and disinfecting skin prior to surgery. FIG. 1 depicts the product delivery device system 100 in an initial configuration, wherein the device 200 and foam applicator 600 are hermetically sealed from the external environment by the package or packaging 800 as discussed above. FIG. 3 depicts the product delivery device 200 in a second configuration where the device 200 is being removed from the package 800 and the product delivery device 200 is in ready-to-use condition without further activation, which is further discussed herein.

Turning back to FIG. 2A, a first end of the product delivery device 200 is configured to be engaged with an individual, such as a patient, whereas a second end of the product delivery device 200 is closest to a user handling the device. The handle 300 can include a first end 301, a second end 302, and a cavity defined therein between the first end and the second end. The handle 300 can have any suitable shape and is depicted as an elongated tubular structure in FIG. 2A. In this embodiment, a radius of the handle is selected to enable the handle to easily rest in and conform to the grip of a user's hand. The handle can be manufactured by any known method, such as but not limited to, injection molding and can be made of any suitable material, such as but not limited to, high density polyethylene, polypropylene, styrene, polyethylene terephthalate, acrylonitrile-butadiene-styrene, or combinations thereof. In other embodiments, the handle itself can be a reusable unit that can be utilized with disposable sterilized foam applicators. Accordingly, such reusable handle can be constructed of a reusable material, such as but not limited to, stainless steel, capable of being autoclaved for reuse with new sterilized foam applicators. The reusable handle can reduce medical waste and provide greater flexibility in selecting the appropriate size of the foam applicator for the clinical procedure to be performed.

As embodied herein, and as depicted in FIG. 2A, the handle 300 can include a flange 307 at the first end 301 of the handle 300. The flange 307 can be used to couple the handle 300 with other components of the product delivery device 200. In some embodiments, the foam applicator pad 600 can be coupled with the flange 307. In other embodiments, the applicator pad 600 can be coupled to a plate member 500 that is coupled to the flange 307, as depicted in FIGS. 1 and 2 and as described further herein.

As shown in FIG. 2A, the handle 300 can also have at least one gripping member such as a rib 303 protruding from the handle 300 to prevent or limit hand slippage toward the first end of the handle during use of the device. The gripping member can be located proximate the second end for ease of holding the device 200. As such, the at least one rib can rest between a user's fingers and can facilitate a grip. Other grips are further contemplated herein, such as but not limited to providing a textured surface to the handle or including other projections for the fingers to rest upon or hold.

The handle of FIG. 2A further includes a sealing member 313 disposed on an external surface of the housing and located between the first end and the second end of the handle. The sealing member 313 can cooperate with the transverse seal to seal the first chamber 805 from the second chamber 810 of the packaging 800. The sealing member 313 acts as a portion of the transverse seal such that the first and second chambers remain isolated in the delivery condition. As such, the handle housed within the second chamber remains dry in the delivery condition. As further explained above, the transverse seal interfaces with the sealing member to provide a hermetic enclosure for the first and second chambers. The sealing member can be any suitable material, such as but not limited to foil, foam, shrink wrap, plastic, adhesive, and other like materials. The sealing member 313 can further include peelable foil packaging, closed-cell plastic foam, gasket or o-ring configurations, ultrasonic bonding, non-peelable films, secondary plastic structures, glue, and a two-shot flexible structure.

As shown in FIG. 2A, the handle 300 further defines a cavity 304 therein, which can store product such as antiseptic solution. In one embodiment, the cavity can include one reservoir to hold product therein, as shown in FIG. 2A. In other embodiments, the handle can define multiple reservoirs to create a plurality of cavities to hold product therein. As such, the cavity of the handle comprises a first reservoir and second reservoir to house a product and a second product respectively. For example, the first reservoir may include an antiseptic solution and the second reservoir may separately include a dye. The volume available for storing product therein can vary depending on the size of the handle. In one embodiment, the handle can hold up to 60 mL of product in the cavity, and in other embodiments up to 1L. The cavity of the handle can have any suitable cross-sectional shape, such as but not limited to a circular cross section, an elliptical cross section, or a rounded triangular cross section.

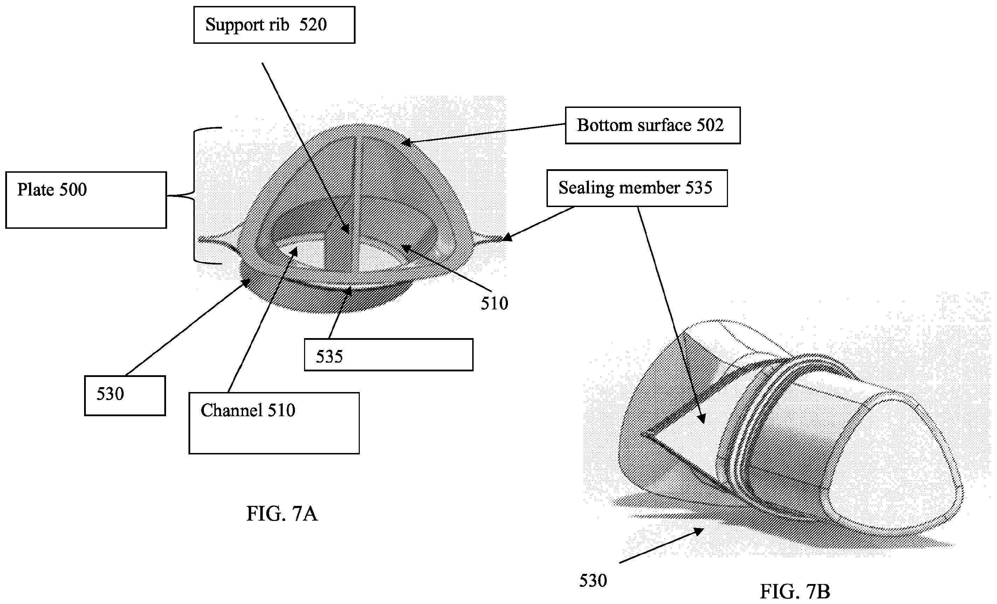

The first end of the handle can further include a plate member 500 to further secure the handle to the foam applicator and/or to facilitate mixing of product when more than one reservoir are defined within the handle. FIG. 7A and 7B depict perspective views of an exemplary plate member 500 of a product delivery device. The plate member can have a bottom surface 502 that interfaces with the flange 307 of the handle and/or interfaces with the foam applicator 600. The plate member can have any suitable size and shape and can have a portion 530 extending from the bottom surface of the plate insertable into the cavity of the handle. The portion 530 can secure the plate to the internal cavity of the handle, as discussed below. The portion 530 can further align channels 510 of the plate member with the reservoirs in the handle. The support rib 520 (and additional ribs as needed) can separate the portion 530 into channels for such alignment with the same number of reservoirs as the handle. The support rib 520 can be selectively disposed within the portion 530 to permit the channels to be fluidly coupled with each other at a location proximate the foam applicator. As such, the plate member can be a distributor configured to mix the solution and the second fluid prior to the respective solution and fluid contacting the foam applicator pad.

In certain applications, the handle can be separated into a reservoir for a first medium and a reservoir for a second medium that are respectively coupled with first and second channels of the portion 530. In such embodiment, the first medium and second mediums can be a gas and/or a flowable medium that is different from each other. In other embodiments, the support rib 520 can extend into the foam applicator pad creating dual application surfaces. The plate member 500 can be manufactured by any known method, such as but not limited to, injection molding and can be made of any suitable material, such as but not limited to, high density polyethylene, polypropylene, styrene, polyethylene terephthalate, acrylonitrile-butadiene-styrene, or combinations thereof.

The bottom surface of the plate 502 can further have a larger surface area than depicted in FIG. 7A and can additionally have a textured surface to facilitate larger surface area for attachment with the applicator foam. Furthermore, the bottom surface can have projections or walls of any suitable size and shape. The walls can provide a raised surface such that the raised surface sidewalls can facilitate a greater surface area so that the foam applicator can better bond to the foam applicator 600 when attached, such as but not limited to a hot plate weld thereto during manufacture, as further described herein. The foam applicator 600 can be coupled to the bottom surface of the plate 502, by any known methods, such as but not limited to, adhesive attachment, ultrasonic and heat welding, and mechanical entanglement.

The plate member 500 can further include a sealing member 535 that can be circumferentially disposed about the portion 530 and plate 500. The sealing member 535 can prevent any product from exiting the handle outside the channels.

As embodied herein, the plate member 500 can be coupled to the handle 300 and the flange 307. The plate member can serve as a connection point for the foam applicator 600 with the flange 307. The plate member 500 can be coupled to the handle using any known methods, including but not limited to, adhesive bonding, threaded connection, interference fit and snap fit.

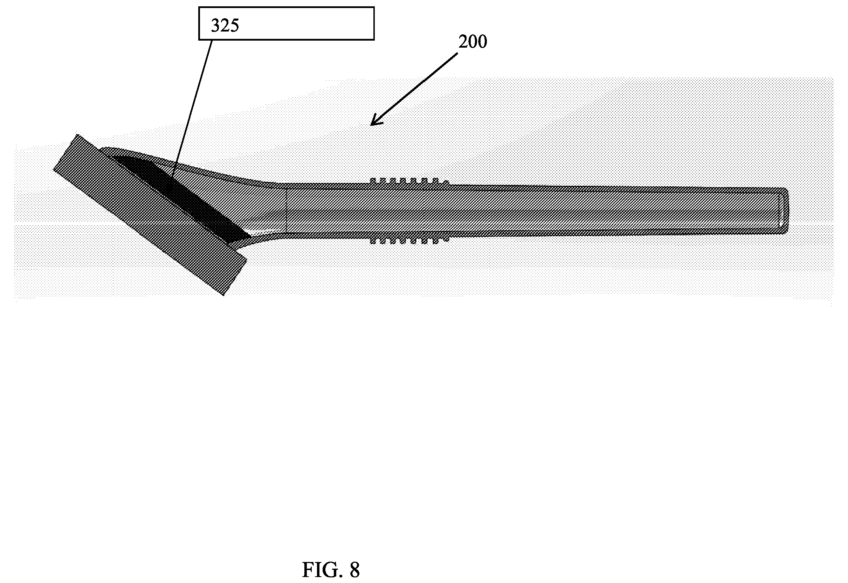

The device can further include additional devices to control the product flow rate from the handle to the foam applicator. In one embodiment and as shown in FIG. 8, the device includes a metering device 325 proximate the first end of the handle, which is configured to release the product in the handle at a predetermined rate. The product delivery can be disposed within the handle and proximate the plate 500. A product delivery can additionally or alternatively be disposed within the foam applicator pad configured to release the product at a predetermined rate. The product delivery can include any suitable device for controlling the flow rate of product exiting the handle, such as but not limited to a porous body with gauged porosity, a thin film with perforations to control the fluid flow, a mechanical regulator that regulates flow by movement or device orientation, felted foam or a surface coating provided on foam to regulate flow, and/or modification of the foam surface porosity by heating or the like. The injecting of the product with the at least one needle as discussed in relation to FIG. 5 and 6 can supplementally provide suitable punctures to a thin film of the device for such metering or the thin film can include perforations prior to injecting for control. The thin film can be disposed at the internal surface of the applicator foam pad.

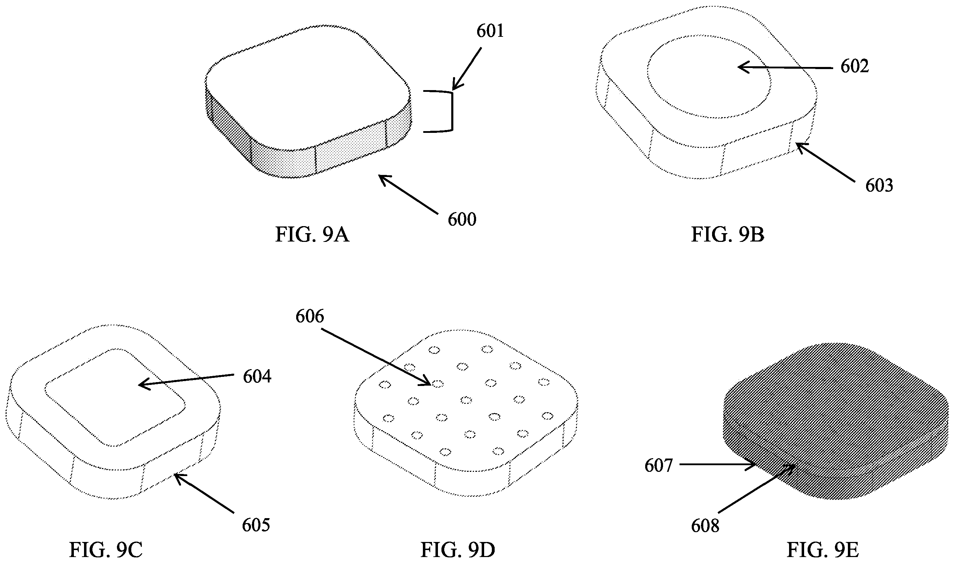

FIGS. 9A-9E depict example embodiments of the foam applicator 600 of the product delivery device 200. Examples of suitable foam applicators are described in U.S. Patent No. 11,160,962. As embodied herein, the foam applicator 600 can function as a liquid reservoir and can store product such as an antiseptic solution until the delivery device is ready for use in addition to storage of the product within the handle. Alternatively, the product can be maintained in the handle of the device until one of the chambers of the packaging 800 is compromised causing the product to exit the handle. In such embodiment, during device assembly, the applicator foam can be delivered in a dry compressed state in the first chamber, which can also be in a compressed state and limited by the perimeter and transverse seal arrangement. When the applicator pad is permitted to contact internal liquid such as through rupturing a breakable seal, the applicator foam is capable of expanding but can be limited by compressed packaging. When the device is released from packaging 800 altogether, the foam can take a full intended form, allowing for foam shapes that are advantageous to the user (e.g. non-parallel wedge). Optionally, the foam applicator pad can be vacuum sealed in the first chamber, which can limit the movement of the product in the handle into the foam applicator pad.

The foam applicator 600 can further function to dispense antiseptic drug product, such as onto a patient's skin. The foam applicator 600 can have any suitable size and shape, and is depicted as a square shape with a thickness dimension and rounded corners as an example. In some embodiments, the foam applicator 600 can be of a size such that the foam applicator 600 extends beyond the plate 500 and the flange 307. The foam applicator 600 can be made of any suitable material for absorbing and dispensing liquids, including but not limited to, reticulated polyester foam, polyurethane foam, a polyester polyurethane blended foam, a polyether polyurethane blended foam, densified foams, felted and non-felted foams, zapped and un zapped foams, clickable foams, laminated foams, reticulated foams, and the like. Methods of manufacturing foam with various shapes, thicknesses, densities, and pore sizes are well known in the art. For purposes of example, and not limitation, an example of a suitable foam is a polyurethane reticulated foam from manufacture FXI (Media, PA), having a range in porosity dimension from approximately 80 pores per linear inch to approximately 110 pores per linear inch in a disclosed embodiment

The material, dimensions, density and pore size of the foam applicator 600 can be selected such that the foam applicator 600 can store a desired amount of liquid antiseptic. In one embodiment, the properties of the foam applicator 600 and the desired amount of liquid antiseptic can further be selected such that the foam applicator can store the entire amount of liquid antiseptic first contained within the handle. In one embodiment, the material, dimensions, density and pore size of the foam applicator 600 can be selected such that the foam applicator 600 can store at least 26 ml of antiseptic solution.

The material, dimensions, density and pore size of the foam applicator 600 can further be selected such that liquid antiseptic does not leak or drip from the foam applicator after the foam applicator 600 is removed from the packaging 800 and prior to patient exposure. In some embodiments, and as depicted in FIG. 9A, the foam applicator 600 can have a monolithic foam structure of consistent density, thickness and pore size. In other embodiments, the foam applicator 600 can consist of two different foam structures that can be passively combined, such as but not limited to an interference fit between the foam structures or can be laminated together. For example and not limitation, the foam applicator can have a square outer foam structure 603 with a circular inner foam structure 602, as shown in FIG. 9B. Alternatively, the foam applicator can have a square outer foam structure 605 with a square inner foam structure 604, as shown in FIG. 9C. However, any desired shapes of the inner foam structure and outer foam structure are contemplated herewith. The foam structures of the foam applicator 600 can be selected to prohibit liquid antiseptic from dripping from the foam applicator 600 after the foam applicator 600 is removed from the packaging 800 and prior to patient exposure. For example and not limitation, the outer foam structure 603 can be selected with a density, thickness and pore size configured to provide superior drip prevention, and inner foam structure 602 can be selected with a different density, thickness and pore size configured to facilitate more ready dispensing of the antiseptic to a patient's skin.

In the embodiment of FIG. 9D, the foam applicator 600 further includes at least one through hole 606, as depicted. The through hole(s) 606 can provide additional channels for the antiseptic solution to travel and release onto the patient's skin. As depicted in FIG. 9E, other embodiments of the foam applicator 600 can have two or more foam structures that can be disposed to create a single foam element with a plurality of layers. The foam layers can be layered using any known methods, including but not limited to, flame lamination, ultrasonic welding, and the like. Foam structures can be selected with different properties including but not limited to, density, thickness and pore size to facilitate fluid retention, superior drip prevention and more ready dispensing of the antiseptic to a patient's skin. For example and not for limitation, the upper foam structure 608 can be selected with a density, thickness and pore size configured to provide superior fluid retention and drip prevention, and lower foam structure 607 can be selected with a different density, thickness and pore size configured to facilitate more ready dispensing of the antiseptic to a patient's skin.

The foam applicator can additionally be impregnated with a medium different than the product located within the cavity during manufacture. For example, the medium can be a dye such that the product in the cavity can transition to the color of the dye when the product exits the handle and contacts the foam.

Once the first and second chambers of the packaging are hermetically sealed, the product can exit the handle to impregnate the foam applicator pad during shipment or otherwise, such that the foam applicator pad becomes pre-saturated in the delivery condition. In another embodiment, the foam applicator pad can additionally be impregnated with the product before the first and second chambers of the packaging are hermetically sealed. As such, the foam applicator pad can hold product therein in addition to having product housed within the handle of the device.

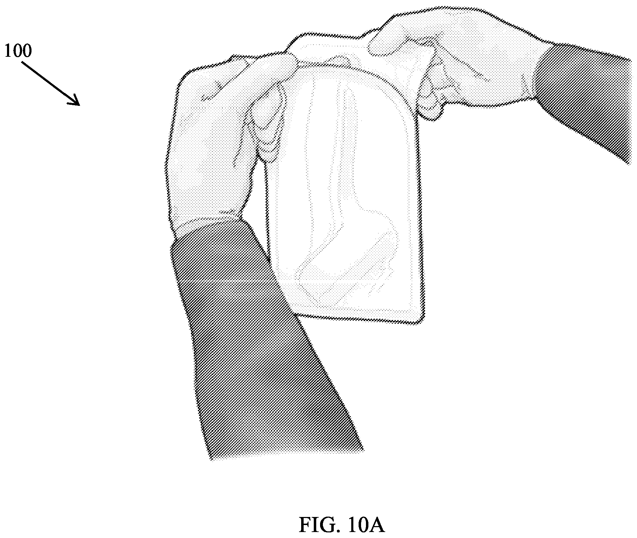

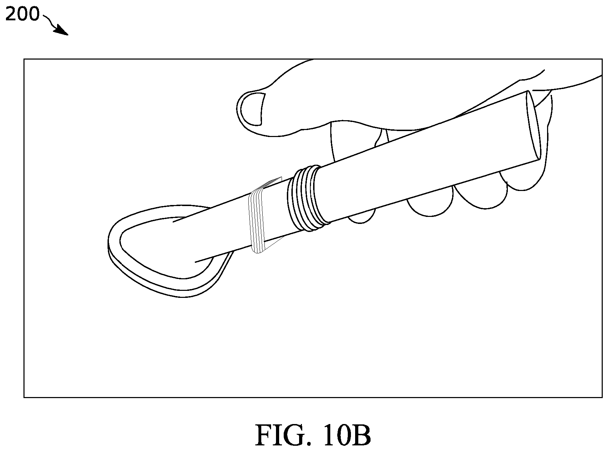

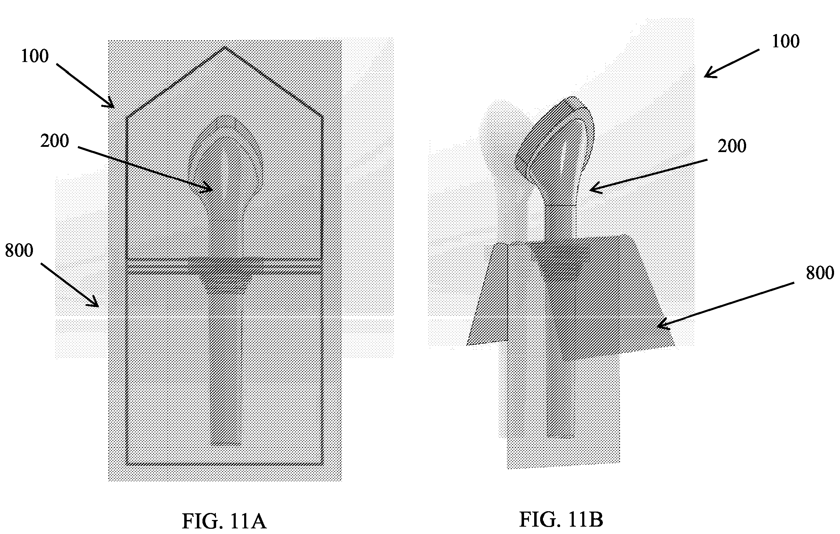

The packaging can be opened by breaking at least a portion of the perimeter seal to access the handle as shown in FIG. 3 and FIG. 10A. The product delivery device can be removed from the packaging using the handle accessible through the second chamber and then the product delivery device 200 can be used, as shown in FIG. 10B. Alternatively, the packaging can be opened by breaking at least a portion of the perimeter seal to access the foam applicator pad in the first chamber while the handle remains sealed in the packaging as represented by FIGS. 11A and 11B.

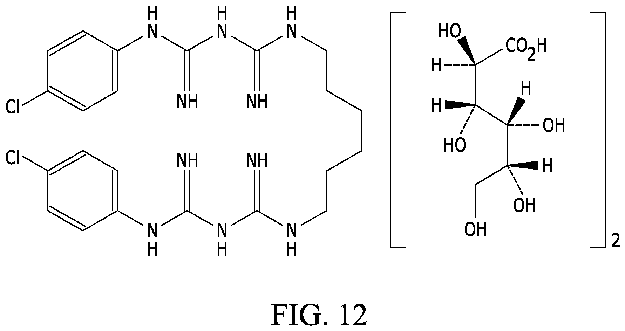

As noted above, the product within the handle can be suitable for any medical application. For instance, the product can be an antiseptic solution, and application of the solution to a portion of a body can kill microorganisms. In one embodiment, application of the antiseptic solution can kill microorganisms immediately and within approximately 10 minutes and further have a persistent effect for at least 7 hours. As such, the antiseptic solution can be used in preparing the body for surgery. In some embodiments, the antiseptic solution can comprise at least one of chlorhexidine gluconate (CHG), isopropyl alcohol, PVP-I, and PVP-IPA, and mixtures thereof including water. In another embodiment, the antiseptic solution can comprise at least 3.15 % w/v chlorhexidine gluconate and 70 % v/v isopropyl alcohol (both ± 10% w/v of nominal value). The CHG can be designated as: 1, 1'---hexamethylenebis [5---(p- --chlorophenyl)biguanide] digluconate, and have the chemical structure shown in FIG. 12.

FIG. 1, FIG. 4C, FIG. 10A and FIG. 11A depict the product delivery device system 100 in an initial configuration wherein the foam applicator 600 is hermetically sealed from the external environment by the packaging 800. As depicted in FIG. 10, the user can grip the packaging 800 at the second end thereof, or at the opposite end for opening as shown in FIG. 11B. As such, the walls of the packaging 800 can be separated to expose the foam applicator 600 to the external environment. Application of this force to the seals can cause the hermetic seal at the perimeter or the transverse seal to breakaway and allow the foam applicator pad 600 to be exposed and ready-to-use without further activation of the device.

The external components of the delivery device 100 can undergo sterilization such as but not limited to ethylene oxide (EtO) sterilization, as practiced in the industry. An outer pouch with a breathable lid stock such as Tyvek from DuPont (Newark, DE) can be used to permit EtO sterilization to ensure external sterility of the delivery device 100 while keeping out other contaminants, such as flora.

The packaging 800 can be made from any suitable material such as but not limited to a relatively flexible resin material, coated papers, shrink wrap, laminate structures, plastic and metalized films and combinations of thereof, calcium carbonate papers or other synthetic paper including various thermoforming packaging solutions.

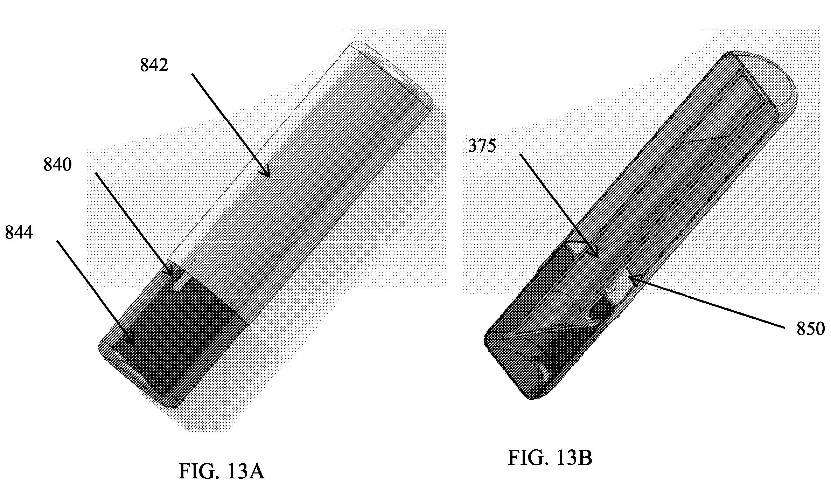

FIGS. 13A and 13B depict an additional embodiment of the disclosed subject matter. In this embodiment, the packaging 800 is embodied as a tube with a first portion 842 and a second portion 844 that respectively define a first chamber and a second chamber therein. The packaging 800 has a pull tab 840 to open the tube, which can be positioned either on the first portion 842 (as shown in FIG. 13A) or alternatively on the second portion 844. The sealing edge 375 of the device interlocks with the bottom portion 850 of the tube to maintain the transverse seal between the first and second chambers. In this embodiment, the sealing edge 375 acts as the sealing member of the handle and can be a gasket or an o-ring. In another embodiment, the first portion 842 can also function as the handle of the delivery device such that the portion 842 is not separable from the delivery device. With such embodiment, the pull tab can be disposed on the second portion 844. In other alternatives, the first and second chambers of the tube can be hermetically sealed from each other, and optionally be sealed to the device to create the seal. In one embodiment, the tube can be extruded and thermoformed to a predetermined shape to house the delivery device. In other embodiments, the tube can be produced in the final form through blow molding or other suitable methods. In further embodiments, The would require reversing which section of the tube has the pull tab.

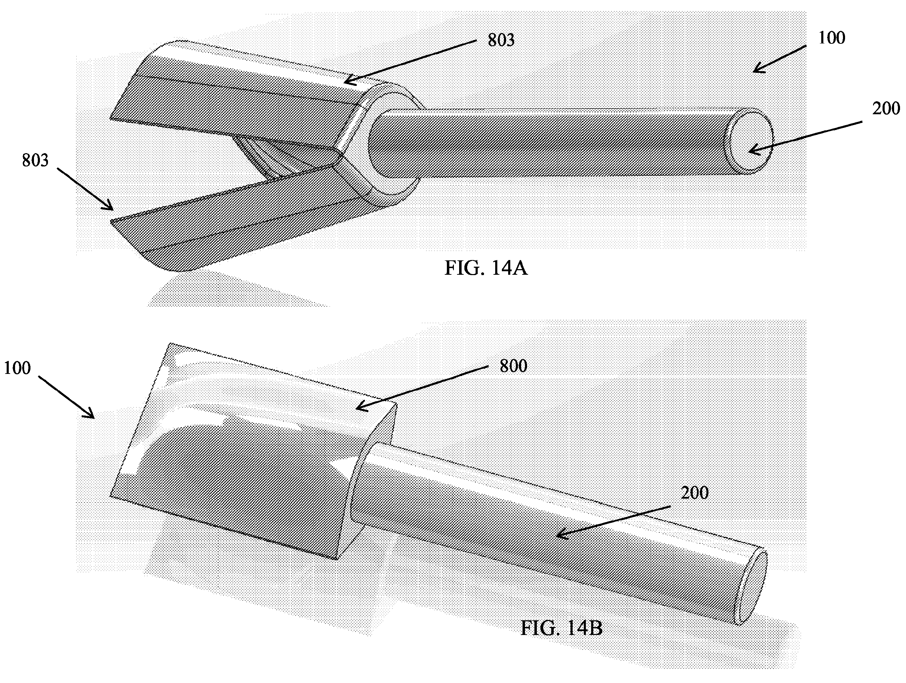

FIGS. 14A and 14B depict an additional embodiment which does not form part of the invention, the packaging 800 is embodied as a single chamber to house the foam applicator pad 600. In this embodiment, the product delivery device and the packaging can be a monolithic unit, as provided in FIG. 14A and 14B. The packaging can include a molded protrusion having arms 803 as depicted in FIG. 14A. The molded protrusion is coupled with the handle of the device. The arms 803 can be folded over the applicator foam pad and sealed to itself to encapsulate the applicator foam pad, as provided in FIG. 14B. During use, the arms can be separated from each other for access to the foam applicator pad therein. Accordingly, the packaging 800 and the device 200 are provided as a single unitary piece. Optionally, an outer housing package can surround the single unitary piece for enhanced sterility.

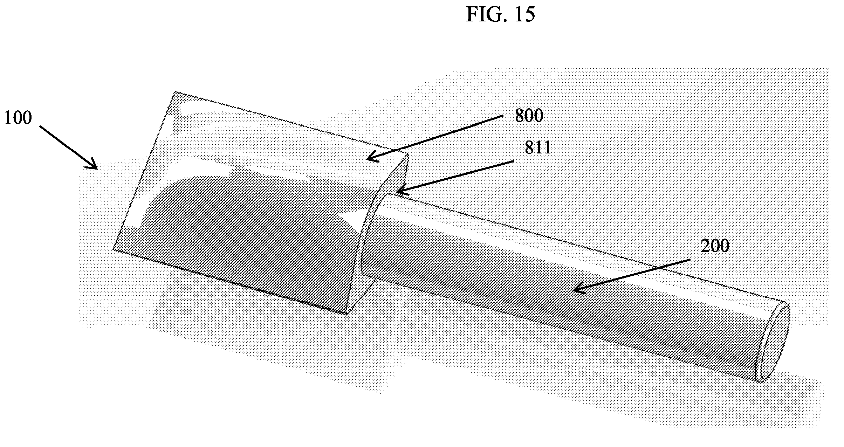

In yet another embodiment which does not form part of the invention, the packaging 800 is embodied as a single chamber to house the foam applicator pad 600, as provided in FIG. 15. In such embodiment, the single chamber can include a blank defining a hole for the handle shaft to be inserted therein. A sealing edge or a sealing member as described herein can interlock the device handle with the packaging at an annular connection 811 to maintain a transverse seal between the single chamber and an external environment. FIG. 15 depicts the packaging 800 as a sealed assembly with the sides and top of the package sealed together.

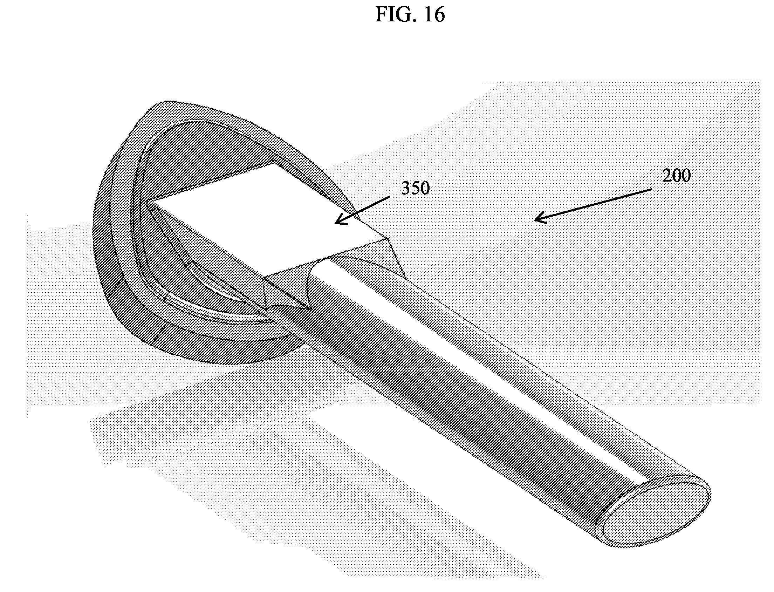

In yet another embodiment which does not form part of the invention, the packaging 800 can comprise a tray member to encapsulate the foam applicator head. An example tray member is described in U.S. Patent No. 11,160,962. The tray member can be manufactured using any suitable technique to facilitate sealing of the tray member to the device, such as blow molding. The tray member can couple with the device in any suitable way, such as heat sealing, adhesives, and the like. The handle of the device can optionally include a gasket 350 to assist with sealing the tray member to the device in embodiments when heat sealing is not utilized for connection, as shown in FIG. 16. A top seal can enclose the applicator using a top surface of the gasket to assist sealing. In this embodiment, the system can be packaged individually or within a kit of devices. In embodiments with a
number of devices as part of a kit, the gasket further can be utilized as part of a seal for an outer housing packaging.

While the disclosed subject matter is described herein in terms of certain embodiments, those skilled in the art will recognize that various modifications and improvements can be made to the disclosed subject matter without departing from the scope of the invention which is defined by the appended claims. Moreover, although individual features of one embodiment of the disclosed subject matter can be discussed herein or shown in the drawings of the one embodiment and not in other embodiments, it should be apparent that individual features of one embodiment can be combined with one or more features of another embodiment or features from a plurality of embodiments.

In addition to the various embodiments depicted and claimed, the disclosed subject matter is also directed to other embodiments having any other possible combination of the features disclosed and claimed herein. As such, the particular features presented herein can be combined with each other in other manners within the scope of the disclosed subject matter such that the disclosed subject matter includes any suitable combination of the features disclosed herein. Thus, the foregoing description of specific embodiments of the disclosed subject matter has been presented for purposes of illustration and description. It is not intended to be exhaustive or to limit the disclosed subject matter to those embodiments disclosed.

It will be apparent to those skilled in the art that various modifications and variations can be made in the device and method of the disclosed subject matter without departing from the scope of the invention which is defined by the appended claims. Thus, it is intended that the disclosed subject matter include modifications and variations that are within the scope of the appended claims.

## Claims

1. A product delivery device system (100), comprising:
a product delivery device (200) having a handle (300) with a first end (301), a second end (302), and a cavity (304) defined therein between the first end (301) and the second end (302),
a product disposed within the cavity (304),
a foam applicator pad (600) coupled to the first end (301) of the handle (300) and in fluid communication with the cavity (304) to receive the product,
**characterized in** the product delivery device system further comprising
a sealing member (313) disposed on an external surface of the handle (300) and located between the first end (301) and the second end (302) of the handle (300); and
a packaging (800) to house the product delivery device (200) therein, the packaging (800) having a first chamber (805) and a second chamber (810) in a delivery condition, wherein the first chamber (805) houses the first end (301) of the handle (300) and the second chamber (810) houses the second end (302) of the handle (300), wherein the first chamber (805) and second chamber (810) are separated by a first breakable hermetic seal (813) and the sealing member (313);
wherein the product delivery device system is configured such that the first end (301) of the handle (300) is isolated in the delivery condition from the second end (302) of the handle (300) to contain the product within the cavity (304) of the handle (300) and the first chamber (805) in the delivery condition;
wherein the first seal (813) is configured to be breakable to release the first end (301) of the handle (300) from the package and the product delivery device is in a ready-to-use condition without further activation.

2. The product delivery device system (100) of claim 1, wherein
i) the foam applicator pad (600) is impregnated with the product; or
ii) the product is an antiseptic solution including at least one of chlorhexidine gluconate, isopropyl alcohol, PVP-I or PVP-IPA; or
iii) the product includes at least one of paint, shoe polish, detergent, or disinfectant.

3. The product delivery device system (100) of claim 1, wherein the foam applicator pad (600) is impregnated with a medium different than the product located within the cavity (304), and optionally wherein the medium is a dye.

4. The product delivery device system (100) of claim 1, wherein the cavity (304) of the handle (300) comprises a first reservoir and second reservoir to house the product and a second fluid, respectively, and optionally wherein the product delivery device system (100) further comprises a distributor, wherein the distributor comprises one or more channels configured to mix the product and the second fluid prior to the respective solution and fluid contacting the foam applicator pad (600), and further optionally wherein the distributor further comprises a support rib (520) which is coupled to the foam applicator (600).

5. The product delivery device system (100) of claim 1, wherein the handle (300) further comprises:
i) a metering device (325) proximate the first end (301), wherein the metering device (325) is configured to release the product at a predetermined rate; or
ii) a gripping member (303) located proximate the second end (302).

6. The product delivery device system (100) of claim 1, wherein
i) the foam applicator pad (600) further comprises a metering device, wherein the metering device is configured to release the solution at a predetermined rate; or
ii) the first chamber (805) and the second chamber (810) are openable independently from each other along a perimeter seal (820).

7. The product delivery device system (100) of claim 1, wherein the first seal (813) is disposed along an interior surface of the packaging (800), and optionally wherein the first seal (813) comprises at least one of glue, adhesive, laminate, or ultrasonic weld.

8. The product delivery device (200) of claim 1, wherein:
i) the product delivery device (200) further comprises a plate (500), having an extension (530) and a bottom surface (502), wherein the extension (530) of the plate (500) is coupled to the first end (301) of the handle (300) and wherein the bottom surface (502) of the plate (500) is coupled to the foam applicator pad (600) to deliver the antiseptic solution from the cavity (304); or
ii) the first chamber (805) and second chamber (810) are separated along the sealing member (313), and wherein the first seal (813) is a transverse seal.

9. A method of manufacturing a product delivery device system comprising:
providing a product delivery device system (100) including
a product delivery device (200) having a handle (300) with a first end (301), a second end (302), and a cavity (304) defined therein between the first end (301) and the second end (302),
a foam applicator pad (600) coupled to the first end (301) of the handle (300) and in fluid communication with the cavity (304) to receive the antiseptic solution,
a sealing member (313), disposed on an external surface of the handle (300), located between the first end (301) and the second end (302) of the handle (300); and providing a packaging (800) to house the product delivery device therein;
forming a second chamber (810) by sealing the second end (302) of the handle (300) with a first breakable seal (813) disposed along the sealing member (313) and along an interior of the packaging (800) and with a segment of a perimeter seal (820);
partially sealing the packaging (800) around the first end (301) of the handle (300) along another segment of the perimeter seal (820) to form a first chamber (805) separate from the second chamber (810), wherein a portion of the first chamber (805) proximate the foam applicator pad (600) is unsealed;
injecting product into the cavity (304) of the handle (300) through the unsealed portion of the first chamber (805); and
sealing the portion of the first chamber (805) proximate the foam applicator pad (600) to hermetically seal the first chamber (805) from the second chamber (810) and an external environment in a delivery condition;
wherein the first chamber (805) is isolated from the second chamber (810) in the delivery condition by the first breakable seal (813) and at least a portion of the perimeter seal (820) and wherein, upon breaking the first breakable seal (813) and at least the portion of the perimeter seal (820), the product delivery device (200) is in a ready-to-use condition without further activation.

10. The method of claim 9, wherein the injecting is performed by using one or more needles which puncture the foam applicator pad (600) to access the cavity (304) of the handle (300).

11. The method of claim 9, further comprising impregnating the foam applicator pad (600) with:
i) the product before the first and second chambers (805, 810) of the packaging (800) are hermetically sealed; or
ii) the fluid after both chambers of the packaging (800) are hermetically sealed.

12. The method of claim 9, further comprising venting air from the cavity (304) of the handle (300) with one or more needles, and optionally wherein the venting of air is conducted simultaneous with the injecting of solution.

13. A method of using a product delivery device system comprising:
providing a product delivery device system (100) including:
a product delivery device (200) in a ready-to-use condition without further activation, the device having a handle (300) with a first end (301), a second end (302), and a cavity (304) defined therein between the first end (301) and the second end (302),
a product disposed within the cavity (304),
a foam applicator pad (600) coupled to the first end (301) of the handle (300) and in fluid communication with the cavity (304) to receive the product,
a sealing member (313), disposed on an external surface of the handle (300), located between the first end (301) and the second end (302) of the handle (300), and
a packaging (800) to house the product delivery device therein, the packaging (800) having a first chamber (805) and a second chamber (810) in a delivery condition, wherein the first chamber (805) houses the first end (301) of the handle (300) and the second chamber (810) houses the second end (302) of the handle (300), wherein the first chamber (805) and second chamber (810) are separated by a first breakable hermetic seal (813) and the sealing member (313), wherein the first end (301) of the handle (300) is isolated in the delivery condition from the second end (302) of the handle (300) to contain the product within the cavity (304) of the handle (300) and the first chamber (805);
breaking the first seal (813) at the sealing member (313) to fluidly couple the first and second chambers (805, 810) of the packaging (800);
releasing at least the first end (301) of the handle (300) from the packaging (800); and
applying the product from product delivery device with the applicator foam pad (600).

14. The method of claim 13, further comprising opening the packaging (800) by breaking a perimeter seal of the second chamber (810) to access the handle (300); and removing the product delivery device from the packaging (800) using the handle (300).

## Patentansprüche

1. Produktausgabevorrichtungssystem (100), Folgendes umfassend:
eine Produktausgabevorrichtung (200), die einen Griff (300) mit einem ersten Ende (301), einem zweiten Ende (302) und einem darin definierten Hohlraum (304) zwischen dem ersten Ende (301) und dem zweiten Ende (302) aufweist,
ein Produkt, das in dem Hohlraum (304) angeordnet ist,
ein Schaumapplikatorkissen (600), das mit dem ersten Ende (301) des Griffs (300) gekoppelt ist und in Fluidverbindung mit dem Hohlraum (304) steht, um das Produkt aufzunehmen, **dadurch gekennzeichnet, dass** das Produktausgabevorrichtungssystem ferner Folgendes umfasst:
ein Dichtungselement (313), das auf einer Außenfläche des Griffs (300) angeordnet ist und sich zwischen dem ersten Ende (301) und dem zweiten Ende (302) des Griffs (300) befindet; und
eine Verpackung (800), um darin die Produktausgabevorrichtung (200) unterzubringen, wobei die Verpackung (800) in einem Ausgabezustand eine erste Kammer (805) und eine zweite Kammer (810) aufweist, wobei die erste Kammer (805) das erste Ende (301) des Griffs (300) unterbringt und die zweite Kammer (810) das zweite Ende (302) des Griffs (300) unterbringt, wobei die erste Kammer (805) und die zweite Kammer (810) durch eine erste brechbare hermetische Dichtung (813) und das Dichtungselement (313) getrennt sind;
wobei das Produktausgabevorrichtungssystem so konfiguriert ist, dass das erste Ende (301) des Griffs (300) in dem Ausgabezustand von dem zweiten Ende (302) des Griffs (300) isoliert ist, um das Produkt in dem Ausgabezustand innerhalb des Hohlraums (304) des Griffs (300) und der ersten Kammer (805) zu halten;
wobei die erste Dichtung (813) so konfiguriert ist, dass sie brechbar ist, um das erste Ende (301) des Griffs (300) von der Verpackung zu lösen, und die Produktausgabevorrichtung ohne weitere Aktivierung in einem gebrauchsfertigen Zustand ist.

2. Produktausgabevorrichtungssystem (100) nach Anspruch 1, wobei
i) das Schaumapplikatorkissen (600) mit dem Produkt imprägniert ist; oder
ii) das Produkt eine antiseptische Lösung ist, die mindestens eines von Chlorhexidingluconat, Isopropylalkohol, PVP-I oder PVP-IPA beinhaltet; oder
iii) das Produkt mindestens eines von Farbe, Schuhcreme, Waschmittel oder Desinfektionsmittel beinhaltet.

3. Produktausgabevorrichtungssystem (100) nach Anspruch 1, wobei das Schaumapplikatorkissen (600) mit einem anderen Medium imprägniert ist als das Produkt, das sich in dem Hohlraum (304) befindet, und optional wobei das Medium ein Farbstoff ist.

4. Produktausgabevorrichtungssystem (100) nach Anspruch 1, wobei der Hohlraum (304) des Griffs (300) einen ersten Behälter und einen zweiten Behälter umfasst, um das Produkt bzw. ein zweites Fluid unterzubringen, und optional wobei das Produktausgabevorrichtungssystem (100) ferner einen Verteiler umfasst, wobei der Verteiler einen oder mehrere Kanäle umfasst, die dazu konfiguriert sind, das Produkt und das zweite Fluid zu mischen, bevor die jeweilige Lösung und das Fluid mit dem Schaumapplikatorkissen (600) in Kontakt kommen, und ferner optional wobei der Verteiler ferner eine Stützrippe (520) umfasst, die mit dem Schaumapplikator (600) gekoppelt ist.

5. Produktausgabevorrichtungssystem (100) nach Anspruch 1, wobei der Griff (300) ferner Folgendes umfasst:
i) eine Dosiervorrichtung (325) in der Nähe des ersten Endes (301), wobei die Dosiervorrichtung (325) dazu konfiguriert ist, das Produkt mit einer vorbestimmten Rate freizusetzen; oder
ii) ein Greifelement (303), das sich in der Nähe des zweiten Endes (302) befindet.

6. Produktausgabevorrichtungssystem (100) nach Anspruch 1, wobei
i) das Schaumapplikatorkissen (600) ferner eine Dosiervorrichtung umfasst, wobei die Dosiervorrichtung dazu konfiguriert ist, die Lösung mit einer vorbestimmten Rate freizusetzen; oder
ii) die erste Kammer (805) und die zweite Kammer (810) unabhängig voneinander entlang einer Umfangsdichtung (820) öffenbar sind.

7. Produktausgabevorrichtungssystem (100) nach Anspruch 1, wobei die erste Dichtung (813) entlang einer Innenfläche der Verpackung (800) angeordnet ist und optional wobei die erste Dichtung (813) mindestens eines von Leim, Klebstoff, Laminat oder Ultraschallschweißung umfasst.

8. Produktausgabevorrichtung (200) nach Anspruch 1, wobei:
i) die Produktausgabevorrichtung (200) ferner eine Platte (500) mit einer Verlängerung (530) und einer Bodenfläche (502) umfasst, wobei die Verlängerung (530) der Platte (500) mit dem ersten Ende (301) des Griffs (300) gekoppelt ist und wobei die Bodenfläche (502) der Platte (500) mit dem Schaumapplikatorkissen (600) gekoppelt ist, um die antiseptische Lösung aus dem Hohlraum (304) auszugeben; oder
ii) die erste Kammer (805) und die zweite Kammer (810) entlang des Dichtungselements (313) getrennt sind und wobei die erste Dichtung (813) eine Querdichtung ist.

9. Verfahren zum Herstellen eines Produktausgabevorrichtungssystems, Folgendes umfassend:
Bereitstellen eines Produktausgabevorrichtungssystems (100), beinhaltend
eine Produktausgabevorrichtung (200), die einen Griff (300) mit einem ersten Ende (301), einem zweiten Ende (302) und einem darin definierten Hohlraum (304) zwischen dem ersten Ende (301) und dem zweiten Ende (302) aufweist,
ein Schaumapplikatorkissen (600), das mit dem ersten Ende (301) des Griffs (300) gekoppelt ist und in Fluidverbindung mit dem Hohlraum (304) steht, um die antiseptische Lösung aufzunehmen,
ein Dichtungselement (313), das an einer Außenfläche des Griffs (300) angeordnet ist und sich zwischen dem ersten Ende (301) und dem zweiten Ende (302) des Griffs (300) befindet; und
Bereitstellen einer Verpackung (800), um darin die Produktausgabevorrichtung unterzubringen;
Bilden einer zweiten Kammer (810) durch Abdichten des zweiten Endes (302) des Griffs (300) mit einer ersten brechbaren Dichtung (813), die entlang des Dichtungselements (313) und entlang eines Innenraums der Verpackung (800) angeordnet ist, und mit einem Segment einer Umfangsdichtung (820);
teilweises Abdichten der Verpackung (800) um das erste Ende (301) des Griffs (300) entlang eines anderen Segments der Umfangsdichtung (820), um eine erste Kammer (805) zu bilden, die von der zweiten Kammer (810) getrennt ist, wobei ein Abschnitt der ersten Kammer (805) in der Nähe des Schaumapplikatorkissens (600) nicht abgedichtet ist;
Injizieren des Produkts in den Hohlraum (304) des Griffs (300) durch den nicht abgedichteten Abschnitt der ersten Kammer (805); und
Abdichten des Abschnitts der ersten Kammer (805) in der Nähe des Schaumapplikatorkissens (600), um die erste Kammer (805) in einem Ausgabezustand hermetisch von der zweiten Kammer (810) und einer äußeren Umgebung abzudichten;
wobei die erste Kammer (805) in dem Ausgabezustand durch die erste brechbare Dichtung (813) und mindestens einen Abschnitt der Umfangsdichtung (820) von der zweiten Kammer (810) isoliert ist und wobei sich die Produktausgabevorrichtung (200) nach dem Brechen der ersten brechbaren Dichtung (813) und mindestens des Abschnitts der Umfangsdichtung (820) ohne weitere Aktivierung in einem gebrauchsfertigen Zustand befindet.

10. Verfahren nach Anspruch 9, wobei das Injizieren unter Verwendung einer oder mehrerer Nadeln durchgeführt wird, die das Schaumapplikatorkissen (600) durchstechen, um auf den Hohlraum (304) des Griffs (300) zuzugreifen.

11. Verfahren nach Anspruch 9, ferner umfassend das Imprägnieren des Schaumapplikatorkissens (600) mit:
i) dem Produkt, bevor die erste und die zweite Kammer (805, 810) der Verpackung (800) hermetisch abgedichtet werden; oder
ii) dem Fluid, nachdem beide Kammern der Verpackung (800) hermetisch abgedichtet sind.

12. Verfahren nach Anspruch 9, ferner umfassend das Ablassen von Luft aus dem Hohlraum (304) des Griffs (300) mit einer oder mehreren Nadeln und optional wobei das Ablassen von Luft gleichzeitig mit dem Injizieren von Lösung durchgeführt wird.

13. Verfahren zum Verwenden eines Produktausgabevorrichtungssystems, Folgendes umfassend:
Bereitstellen eines Produktausgabevorrichtungssystems (100), Folgendes beinhaltend:
eine Produktausgabevorrichtung (200) in einem gebrauchsfertigen Zustand ohne weitere Aktivierung, wobei die Vorrichtung einen Griff (300) mit einem ersten Ende (301), einem zweiten Ende (302) und einem darin definierten Hohlraum (304) zwischen dem ersten Ende (301) und dem zweiten Ende (302) aufweist,
ein Produkt, das in dem Hohlraum (304) angeordnet ist,
ein Schaumapplikatorkissen (600), das mit dem ersten Ende (301) des Griffs (300) gekoppelt ist und in Fluidverbindung mit dem Hohlraum (304) steht, um das Produkt aufzunehmen,
ein Dichtungselement (313), das an einer Außenfläche des Griffs (300) angeordnet ist und sich zwischen dem ersten Ende (301) und dem zweiten Ende (302) des Griffs (300) befindet, und
eine Verpackung (800), um darin die Produktausgabevorrichtung unterzubringen, wobei die Verpackung (800) eine erste Kammer (805) und eine zweite Kammer (810) in einem Ausgabezustand aufweist, wobei die erste Kammer (805) das erste Ende (301) des Griffs (300) unterbringt und die zweite Kammer (810) das zweite Ende (302) des Griffs (300) unterbringt, wobei die erste Kammer (805) und die zweite Kammer (810) durch eine erste brechbare hermetische Dichtung (813) und das Dichtungselement (313) getrennt sind, wobei das erste Ende (301) des Griffs (300) in dem Ausgabezustand von dem zweiten Ende (302) des Griffs (300) isoliert ist, um das Produkt innerhalb des Hohlraums (304) des Griffs (300) und der ersten Kammer (805) zu halten;
Brechen der ersten Dichtung (813) an dem Dichtungselement (313), um die erste und die zweite Kammer (805, 810) der Verpackung (800) fluidisch zu koppeln;
Lösen mindestens des ersten Endes (301) des Griffs (300) von der Verpackung (800); und
Applizieren des Produkts von der Produktausgabevorrichtung mit dem Applikatorschaumkissen (600) .

14. Verfahren nach Anspruch 13, ferner umfassend ein Öffnen der Verpackung (800) durch Brechen einer Umfangsdichtung der zweiten Kammer (810), um auf den Griff (300) zuzugreifen; und ein Entfernen der Produktausgabevorrichtung aus der Verpackung (800) unter Verwendung des Griffs (300).

## Revendications

1. Système de dispositif de distribution de produit (100), comprenant :
un dispositif de distribution de produit (200) comportant une poignée (300) avec une première extrémité (301), une deuxième extrémité (302) et une cavité (304) définie entre la première extrémité (301) et la deuxième extrémité (302),
un produit disposé dans la cavité (304),
un tampon applicateur en mousse (600) fixé à la première extrémité (301) de la poignée (300) et en communication fluidique avec la cavité (304) pour recevoir le produit,
**caractérisé en ce que** le système de dispositif de distribution de produit comprenant en outre
un élément d'étanchéité (313) disposé sur une surface externe de la poignée (300) et situé entre la première extrémité (301) et la deuxième extrémité (302) de la poignée (300) ; et
un emballage (800) destiné à contenir le dispositif de distribution de produit (200), l'emballage (800) ayant une première chambre (805) et une deuxième chambre (810) en condition de distribution, dans lequel la première chambre (805) abrite la première extrémité (301) de la poignée (300) et la deuxième chambre (810) abrite la deuxième extrémité (302) de la poignée (300), dans lequel la première chambre (805) et la deuxième chambre (810) sont séparées par un premier joint hermétique cassable (813) et l'élément d'étanchéité (313) ;
dans lequel le système de dispositif de distribution de produit est configuré de telle sorte que la première extrémité (301) de la poignée (300) soit isolée dans l'état de distribution de la deuxième extrémité (302) de la poignée (300) pour contenir le produit dans la cavité (304) de la poignée (300) et la première chambre (805) dans l'état de distribution ;
dans lequel le premier joint (813) est configuré pour être cassable afin de libérer la première extrémité (301) de la poignée (300) de l'emballage et le dispositif de distribution du produit est prêt à l'emploi sans activation supplémentaire.

2. Système de dispositif de distribution de produit (100) selon la revendication 1, dans lequel
i) le tampon applicateur en mousse (600) est imprégné du produit ; ou
ii) le produit est une solution antiseptique contenant au moins un parmi le gluconate de chlorhexidine, l'alcool isopropylique, PVP-I ou PVP-IPA ; ou
iii) le produit comprend au moins un parmi une peinture, un cirage, un détergent ou un désinfectant.

3. Système de dispositif de distribution de produit (100) selon la revendication 1, dans lequel le tampon applicateur en mousse (600) est imprégné d'un milieu différent du produit situé dans la cavité (304), et en éventuellement dans lequel le milieu est un colorant.

4. Système de dispositif de distribution de produit (100) selon la revendication 1, dans lequel la cavité (304) de la poignée (300) comprend un premier réservoir et un deuxième réservoir destinés à contenir respectivement le produit et un deuxième fluide, et, en éventuellement, dans lequel le système de dispositif de distribution de produit (100) comprend en outre un distributeur, dans lequel le distributeur comprend un ou plusieurs canaux configurés pour mélanger le produit et le deuxième fluide avant que la solution et le fluide respectifs n'entrent en contact avec le tampon applicateur en mousse (600), et, éventuellement, dans lequel le distributeur comprend en outre une nervure de support (520) qui est couplée à l'applicateur en mousse (600).

5. Système de dispositif de distribution de produit (100) selon la revendication 1, dans lequel la poignée (300) comprend en outre :
i) un dispositif de dosage (325) situé à proximité de la première extrémité (301), dans lequel le dispositif de dosage (325) est configuré pour libérer le produit à un débit prédéterminé ; ou
ii) un élément de préhension (303) situé à proximité de la deuxième extrémité (302).

6. Système de dispositif de distribution de produit (100) selon la revendication 1, dans lequel
i) le tampon applicateur en mousse (600) comprend en outre un dispositif de dosage, dans lequel le dispositif de dosage est configuré pour libérer la solution à un débit prédéterminé ; ou
ii) la première chambre (805) et la deuxième chambre (810) peuvent être ouvertes indépendamment l'une de l'autre le long d'un joint périmétrique (820).

7. Système de dispositif de distribution de produit (100) selon la revendication 1, dans lequel le premier joint (813) est disposé le long d'une surface intérieure de l'emballage (800), et éventuellement dans lequel le premier joint (813) comprend au moins une colle, un adhésif, un stratifié ou une soudure ultrasonique.

8. Dispositif de distribution de produit (200) selon la revendication 1, dans lequel :
i) le dispositif d'administration du produit (200) comprend en outre une plaque (500), comportant une extension (530) et une surface inférieure (502), l'extension (530) de la plaque (500) étant fixée à la première extrémité (301) de la poignée (300) et la surface inférieure (502) de la plaque (500) étant fixée au tampon applicateur en mousse (600) pour distribuer la solution antiseptique à partir de la cavité (304) ; ou
ii) la première chambre (805) et la deuxième chambre (810) sont séparées le long de l'élément d'étanchéité (313), et dans lequel le premier joint (813) est un joint transversal.

9. Procédé de fabrication d'un système de dispositif de distribution de produit comprenant :
la fourniture d'un système de dispositif de distribution de produit (100) comprenant
un dispositif de distribution de produit (200) comportant une poignée (300) avec une première extrémité (301), une deuxième extrémité (302) et une cavité (304) définie entre la première extrémité (301) et la deuxième extrémité (302),
un tampon applicateur en mousse (600) couplé à la première extrémité (301) de la poignée (300) et en communication fluidique avec la cavité (304) pour recevoir la solution antiseptique,
un élément d'étanchéité (313), disposé sur une surface externe de la poignée (300), situé entre la première extrémité (301) et la deuxième extrémité (302) de la poignée (300) ; et
la fourniture d'un emballage (800) destiné à contenir le dispositif de distribution de produit ;
la formation d'une deuxième chambre (810) en scellant la deuxième extrémité (302) de la poignée (300) avec un premier joint cassable (813) disposé le long de l'élément de joint (313) et le long d'un intérieur de l'emballage (800) et avec un segment d'un joint périmétrique (820) ;
le scellement partiel de l'emballage (800) autour de la première extrémité (301) de la poignée (300) le long d'un autre segment du joint périmétrique (820) pour former une première chambre (805) séparée de la deuxième chambre (810), dans lequel une partie de la première chambre (805) proche du tampon applicateur en mousse (600) est non scellée ;
l'injection du produit dans la cavité (304) de la poignée (300) à travers la partie non scellée de la première chambre (805) ; et
le scellement de la partie de la première chambre (805) proche du tampon applicateur en mousse (600) pour sceller hermétiquement la première chambre (805) de la deuxième chambre (810) et d'un environnement extérieur dans une condition de distribution ;
dans lequel la première chambre (805) est isolée de la deuxième chambre (810) dans l'état de distribution par le premier joint cassable (813) et au moins une partie du joint périmétrique (820) et dans lequel, lors de la rupture du premier joint cassable (813) et de l'au moins une partie du joint périmétrique (820), le dispositif de distribution de produit (200) est prêt à l'emploi sans autre activation.

10. Procédé selon la revendication 9, dans lequel l'injection est réalisée à l'aide d'une ou plusieurs aiguilles qui percent le tampon applicateur en mousse (600) pour accéder à la cavité (304) de la poignée (300).

11. Procédé selon la revendication 9, comprenant en outre l'imprégnation du tampon applicateur en mousse (600) avec :
i) le produit avant que les première et deuxième chambres (805, 810) de l'emballage (800) ne soient scellées hermétiquement ; ou
ii) le fluide après que les deux chambres de l'emballage (800) soient scellées hermétiquement.

12. Procédé selon la revendication 9, comprenant en outre l'évacuation de l'air de la cavité (304) de la poignée (300) à l'aide d'une ou plusieurs aiguilles, et éventuellement dans lequel l'évacuation de l'air est effectuée simultanément à l'injection de la solution.

13. Procédé d'utilisation d'un système de dispositif de distribution de produit comprenant :
la fourniture d'un système de dispositif de distribution de produit (100) comprenant :
un dispositif de distribution de produit (200) en état prêt à l'emploi sans activation supplémentaire, le dispositif ayant une poignée (300) avec une première extrémité (301), une deuxième extrémité (302) et une cavité (304) définie entre la première extrémité (301) et la deuxième extrémité (302),
un produit disposé dans la cavité (304),
un tampon applicateur en mousse (600) fixé à la première extrémité (301) de la poignée (300) et en communication fluidique avec la cavité (304) pour recevoir le produit,
un élément d'étanchéité (313), disposé sur une surface externe de la poignée (300), situé entre la première extrémité (301) et la deuxième extrémité (302) de la poignée (300), et
un emballage (800) destiné à contenir le dispositif de distribution de produit, l'emballage (800) ayant une première chambre (805) et une deuxième chambre (810) en condition de distribution, la première chambre (805) abritant la première extrémité (301) de la poignée (300) et la deuxième chambre (810) abritant la deuxième extrémité (302) de la poignée (300), la première chambre (805) et la deuxième chambre (810) étant séparées par un premier joint hermétique cassable (813) et l'élément d'étanchéité (313), la première extrémité (301) de la poignée (300) étant isolée en condition de distribution de la deuxième extrémité (302) de la poignée (300) pour contenir le produit dans la cavité (304) de la poignée (300) et la première chambre (805) ;
la rupture du premier joint (813) au niveau de l'élément d'étanchéité (313) pour coupler de manière fluide les première et deuxième chambres (805, 810) de l'emballage (800) ;
la libération d'au moins la première extrémité (301) de la poignée (300) de l'emballage (800) ; et
l'application du produit à partir du dispositif de distribution de produit avec le tampon applicateur en mousse (600).

14. Procédé selon la revendication 13, comprenant en outre l'ouverture de l'emballage (800) en rompant un joint périmétrique de la deuxième chambre (810) pour accéder à la poignée (300) ; et le retrait du dispositif de distribution de produit de l'emballage (800) à l'aide de la poignée (300).
